# EUROPEAN PATENT SPECIFICATION

(11) **EP 2 255 712 B1**
(45) Date of publication and mention of the grant of the patent: **26.12.2012**
(21) Application number: 10162977.2
(22) Date of filing: 17.05.2010
(51) Int. Cl.: A47L 13/20

(54) **Telescopically rotatable mop**
Teleskopisch drehbarer Mopp
Serpillière pouvant tourner téléscopiquement

(30) Priority: 27.05.2009 TW 098209417 U
(43) Date of publication of application: 01.12.2010
(73) Proprietor: Tuo Shen International Corporation Limited, Guishan Township T'ao yuan 333 (TW)
(72) Inventor: Chen, Yung-Hua, 247 Taipei County (TW)
(74) Representative: Chaillot, Geneviève

(56) References cited:
- DE-A1-102005 023 084
- GB-A- 235 684

## Description

The invention relates to a telescopically rotatable mop, and more particularly to a structure that ensures a smooth operation in dewatering the mop with one hand only and without use of the feet.

After a mop has been used, it is necessary to wring dirty water from mop fabrics (or cotton strips) of the mop before soaking clean water again to facilitate washing a floor, and mopping is obviously a tiresome job. Therefore, related manufacturers have developed various different dewatering devices for the mop, such as a dewatering device disclosed in TW M347146U, wherein a pedal is provided for driving a gear to rotate a dewatering tank at a fast speed, so as to wring cotton strips of the mop placed in the dewatering tank. Although the aforementioned device can improve the inconvenient way of wringing the mop fabrics by hands, yet the operation still requires a user to step on the pedal continuously by one foot, and keep the user's body in balance by another foot. Such arrangement not only involves an inconvenient operation, but also endangers the safety of users when the users fail to stand stably or fall. Therefore, it is necessary to develop a mop with an easy, convenient and safe operation in dewatering.

DE 10 2005 023 084 A1 discloses a telescopically rotatable mop comprising a hollow rod with an inside thread coupled to a stranded rod via a freewheel such that linear axial movement of the hollow rod propels the stranded rod in a single direction of rotation, a disc mop being coupled to the other end of the stranded rod to be rotated therewith, and a locking device for locking the hollow rod against the stranded rod.

The object of the invention is to provide a telescopically rotatable mop that permits a convenient operation with less effort when the internal and external rods rotate in a telescopic way. In this way, the operation failure may be minimized and the service life may be increased.

In order to achieve the above-mentioned object, the invention includes a telescopically movable mop according to claim 1.

Accordingly, the actuating element is rotated by a linear motion of the driving element when the external rod is moved up-and-down. Moreover, the engaging element is driven in rotation in one direction only, thereby creating a continuous rotation of the internal rod and the disc body in the same direction by the inertia force. As a result, a centrifugal force is produced to throw away the water absorbed in the mop yarns.

The accomplishment of this and other objects of the invention will become apparent from the following descriptions and its accompanying figures of which:
FIG. 1 is a perspective view of the invention;
FIG. 2 is an exploded perspective view of the invention;
FIG. 3 is an exploded perspective view of the main structure of the invention with the engaging element illustrated in half section.
FIG. 4 is an exploded perspective view of the main structure of the invention with the engaging element and the annular element in the connection position
FIG. 5 is a cross-sectional view of the main structure of the invention with the internal and external rods in a position of relative motion;
FIG. 6 is a cross-sectional view taken along the line 6-6 in FIG. 5;
FIG. 7 is a cross-sectional view of the structure in accordance with the invention, showing that the external rod is pressed downward;
FIG. 8 is a cross-sectional view of the structure in accordance with the invention, showing that the external rod is pulled upward;
FIG. 9 is a schematic drawing of the locking mechanism of the invention in a loosened position when the external clamping sleeve is lifted;
FIG. 10 is a schematic drawing of the locking mechanism of the invention in a tightened position when the external clamping sleeve is lowered;
FIG. 11 is an application view I of the invention; and
FIG. 12 is an application view II of the invention.

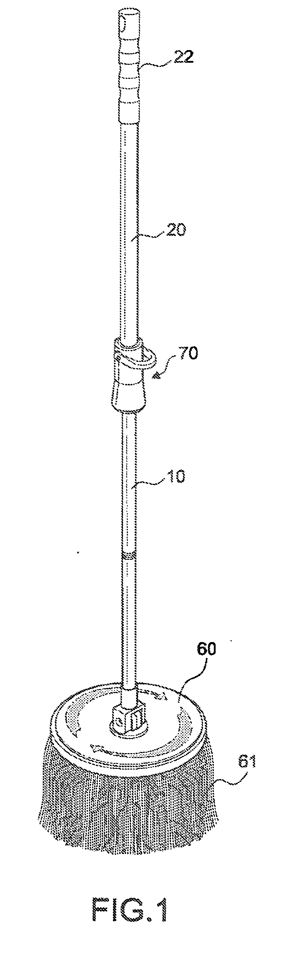

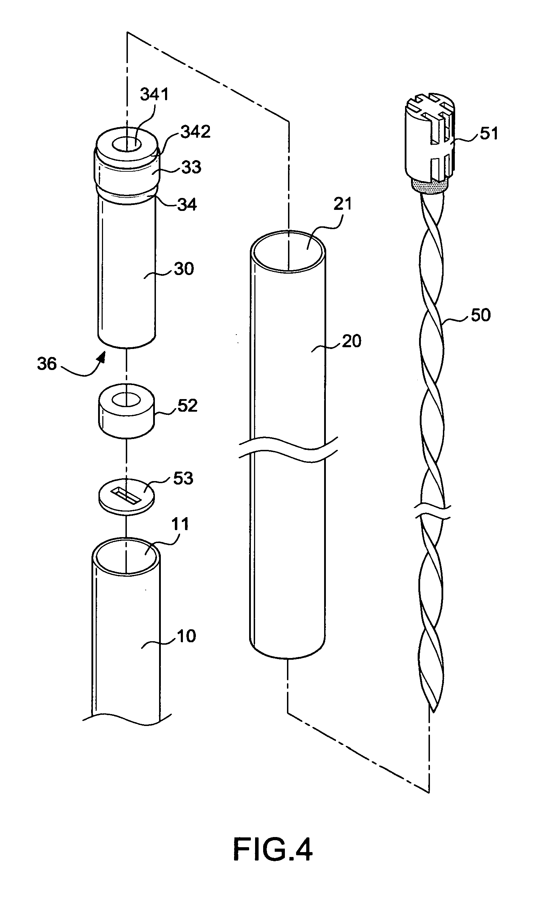

First of all, referring to FIGS. 1 through 8, a mop in accordance with the invention includes an internal rod 10, an external rod 20, an engaging element 30, a driving element 50, an actuating element 40, a disc body 60 and a locking mechanism 70.

The internal rod 10 is constructed as a hollow circular tube and made by metal or non-metal material. Therefore, it can be an aluminum tube or a plastic tube.

The external rod 20 includes a bottom portion in a telescopic connection with a top portion of the internal rod 10. According to the embodiment, the operator can hold on the external rod 20 to conduct a telescopic motion on the internal rod 10.

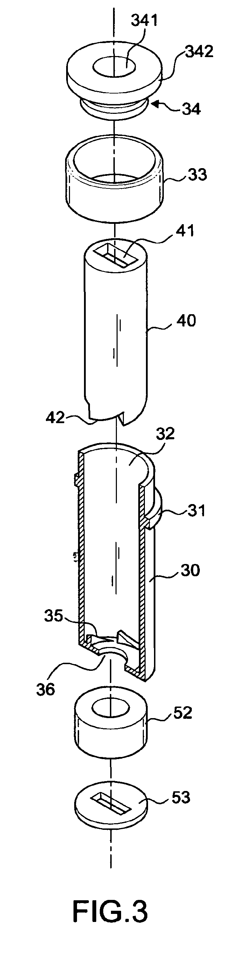

The engaging element 30 is positioned within the opening 11 at the top of the internal rod 10. According to this embodiment, an annular element 33 and a fixing cap 34 are mounted and fixed on the engaging element 30 after the engaging element 30 is placed within the top of the internal rod 10. The upper portion of the engaging element 30 is externally provided with a flange 31. The fixing cap 34 includes a through hole 341 at the top thereof and a projecting flange 342 at the external rim thereof. The bottom of the fixing cap 34 fits into an opening 32 of the engaging element 30 in place. As shown in FIG. 3, the bottom of the internal rim of the engaging element 30 is provided with driven teeth 35.

The driving element 50 is formed in an elongated shape and positioned within the external rod 20 in such a way that the driving element 50 is moved up and down synchronically with the external rod 20. According to the embodiment, the driving element 50 includes a fixing block 51 fastened by a fixing element (not shown) or in a riveting way within the top end 21 of the external rod 20. Moreover, a protection sleeve 22 is mounted on the external rod 20.

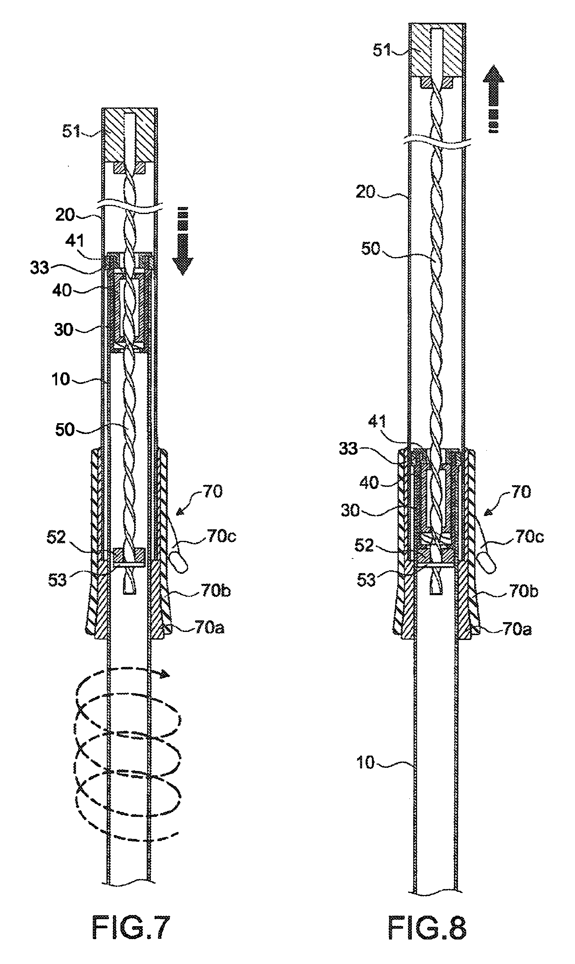

The actuating element 40 is positioned within the engaging element 30 for accommodating the driving element 50. The driving element 50 is constructed as a threaded piece. As a result, the internal wall of the actuating element 40 has to be formed to be a threaded sleeve 41. According to the structure of the worm or the threaded piece, the actuating element 40 is correspondingly provided with a worm thread or an elongated groove such that the driving element 50 may impart a rotary motion to the actuating element 40 by means of the up-and-down linear movement of the external rod 20. According to this embodiment, the driving element 50 is constructed as a threaded piece. As a result, the threaded sleeve 41 at the internal end of the actuating element 40 is constructed as an elongated groove such that the up-and-down movement of the driving element 50 in the threaded sleeve 41 may impart a rotary motion to the actuating element 40 within the engaging element 30. As shown in FIG. 3, the bottom of the actuating element 40 is provided with downward driving teeth 42 in contract with the upward driven teeth 35 of the engaging element 30. Since the engaging teeth 35, 42 are formed in an inclined way, the drive is subject to a rotation in a certain direction. As shown in FIG. 7, the engaging element 30 is subject to a clockwise rotation like the actuating element 40 when the actuating element 40 is driven by the driving element 50. In this way, the actuating element 40 is driven when the driving element 50 is compressed downward. Meanwhile, the engaging element 30 is brought in clockwise rotation. To the contrary, as shown in FIG. 8, when the driving element 50 is pulled upward, the actuating element 40 is brought in a counterclockwise rotation. At that time, the downward driving teeth 42 of the actuating element 40 are driven in an idle non-rotation state relative to the driven teeth 35 of the engaging element 30. In other words, the engaging element 30 remains unmoved such that the driving element 50 can be returned to the original position for a renewed downward pression to drive the engaging element 30 again.

The disc body 60 is secured to the bottom of the internal rod 10 and includes mop yarns 61.

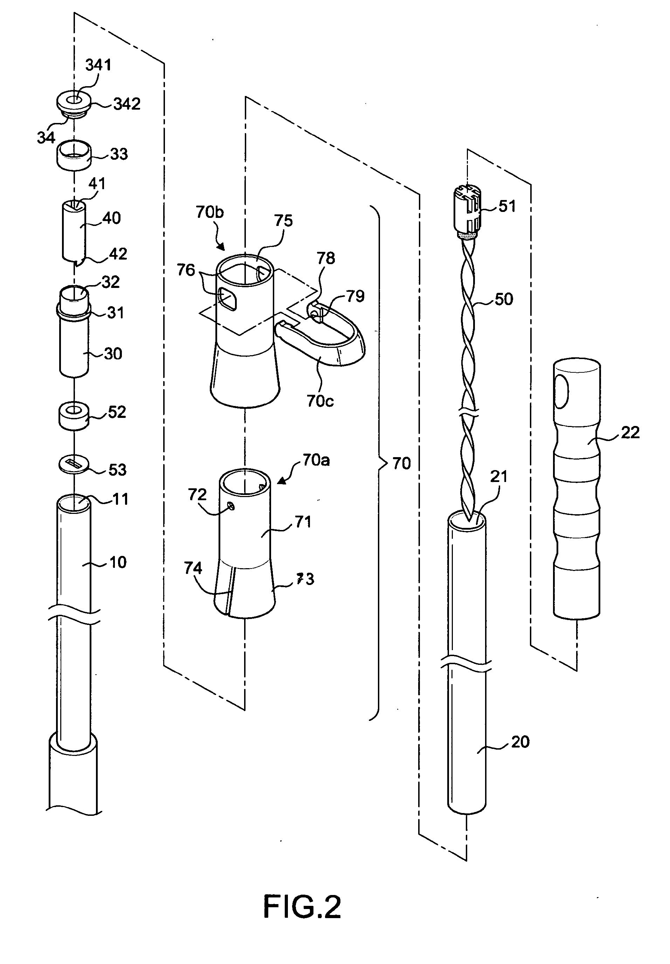

The locking mechanism 70 is mounted on the external rod 20 for locking the internal rod 10 and the external rod 20 in place or for unlocking them in a telescopic state. As shown in FIGS. 2, 9 and 10, the locking mechanism 70 includes an internal clamping sleeve 70a, an external clamping sleeve 70b and a U-shaped lever 70c, but should not be limited thereto.
The internal clamping sleeve 70a includes an internal tube 71 at the top thereof. The bottom of the external rod 20 is introduced into the internal tube 71 and fastened there in place. The fastening effect may be achieved in the clamping, locking, hooking or screwing way. The fastening technique belongs to the prior art so that no further descriptions thereto are given hereinafter. Both sides of the internal clamping sleeve 70a are provided with positioning holes 72. Moreover, the bottom portion of the internal clamping sleeve 70a is constructed as a conic body 73 (extending or expanding from the top to the bottom), with an indentation 74. The indentation 74 is extended in axial direction. Preferably, there are at least two indentations 74.

The external clamping sleeve 70b is mounted on the periphery of the internal clamping sleeve 70a. The upper portion of the external clamping sleeve 70b is provided with an external tube 75 corresponding to the internal tube 71. The external tube 75 includes at both sides thereof two mounting holes 76 in aligmnent with the positioning holes 72 of the internal tube 71. According to the embodiment, the mounting holes 76 are formed as non-circular and rectangular holes. The mounting holes 76 and the positioning holes 72 are not concentrically position, such that cams 78 within the mounting holes 76 tend to conduct an eccentric push action. A bell mouth 77 is formed at the lower portion of the external clamping sleeve 70b for fitting over the conic body 73.

The U-shaped lever 70c includes a swivel protrusion 79 and the eccentric cam 78 at the internal wall of both sides thereof for fitting into the positioning holes 72 of the internal clamping sleeve 70a and the mounting holes 76 of the external clamping sleeve 70b. Besides, the eccentric cams 78 are positioned within the mounting holes 76. According to this embodiment, the swivel protrusions 79 together with the eccentric cams 78 and the U-shaped lever arm 70c are formed by the injection-molding process. However, it should not be restricted thereto. In other words, the swivel protrusion 79 can be replaced by a processed metal post.

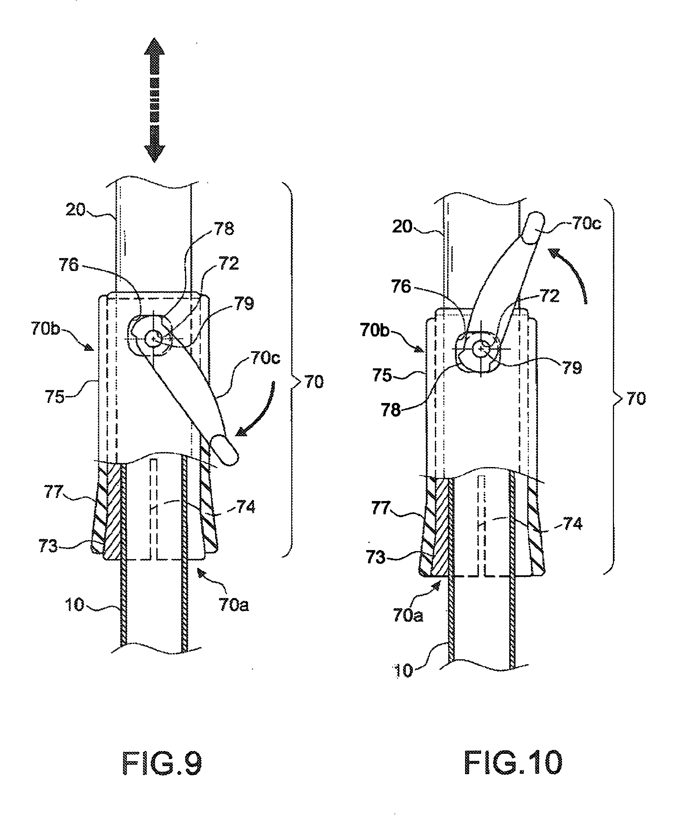

Based upon the above-mentioned structure, when the U-shaped lever 70c swivels on the swivel protrusions 79, the eccentric cams 78 are offset within the mounting holes 76, thereby moving the external clamping sleeve 70b on the periphery of the internal clamping sleeve 70a upward or downward. As shown in FIG. 9, the external rim of the internal clamping sleeve 70a rises when the U-shaped lever 70c is pushed downward. Due to the action of the indentation 74, the bell mouth 77 of the external clamping sleeve 70b is brought in a loosened position relative to the conic body 73 of the internal clamping sleeve 70a. As a result, the internal rod 10 and the external rod 20 are unlocked and brought in a telescopic state. As shown in FIG. 10, the external clamping sleeve 70b is moved downward when the U-shaped lever 70c is pulled upward. In this way, the internal clamping sleeve 70a is so clamped that the internal and external rods 10, 20 are fixed in place.

As shown in FIG. 7, when the locking mechanism 70 is unlocked in an opened position and the external rod 20 is pressed downward, the driving element 50 is synchronically lowered to pass through the threaded sleeve 41 of the actuating element 40. In this way, the actuating element 40 is rotated clockwise so as to cause a synchronic rotation of the engaging element 30. The engaging element 30 is tightly secured to the internal rod 10. Therefore, the internal rod 10 tends to be rotated in a single direction. When the external rod 20 is pulled upward, the actuating element 40 is in an idle state relative to the engaging element 30 when rotated counterclockwise (see FIG. 8). In this way, the internal rod 10 is subject to a continuous rotation in a clockwise direction due to the inertia force without any intervention from the upward pull of the external rod 20.

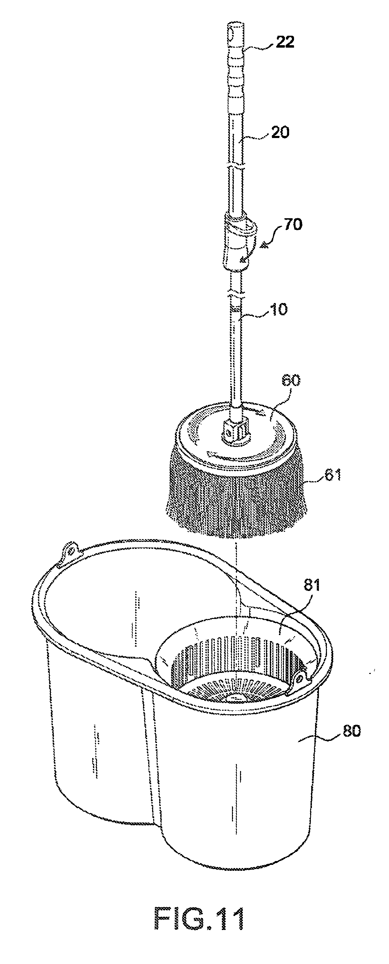

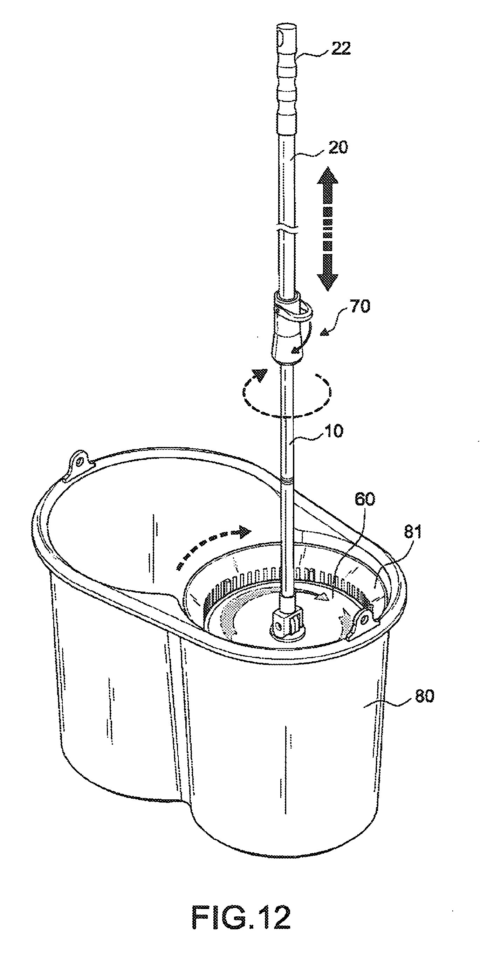

As shown in FIGS. 11 and 12, when the external rod 20 is pushed downward, the internal rod 10 and the disc body 60 are rotated in a single direction, thereby moving the mop yarns 61 (see FIG. 11) attached to the disc body 60 by the centrifugal force outward.

Furthermore, when the external rod 20 is pulled upward, as depicted above, the internal rod 10 won't be acted upon thereby and remains to rotate in the same direction due to the action of the inertia force. In this way, the internal rod 10 and the disc body 60 may be rotated more than 10 times within a dewatering basket 81 of a bucket body 80 by means that the user pushes downward and pulls upward the external rod 20 for a few times. Unlike a conventional bucket body employing an internal drive mechanism to drive its dewatering basket in rotation by a user's foot, the dewatering basket 81 according to this embodiment is rotatable within the bucket body 80. Unlike the conventional way, the dewatering basket 81 in accordance with the invention may be synchronically driven in rotation when the disc body 60 is rotated by the internal rod 10. In this way, the mop yarns 61 of the disc body 60 are subject to the centrifugal force for dewatering. Meanwhile, the water removed may be received within the bucket body 80.

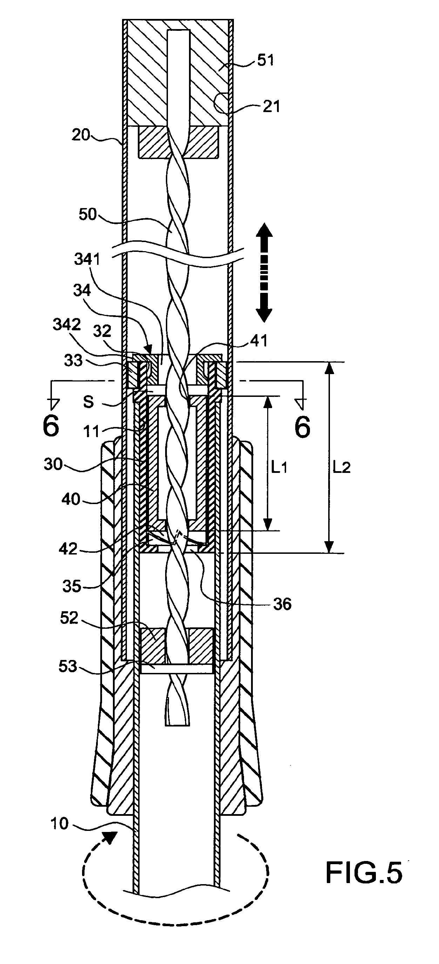

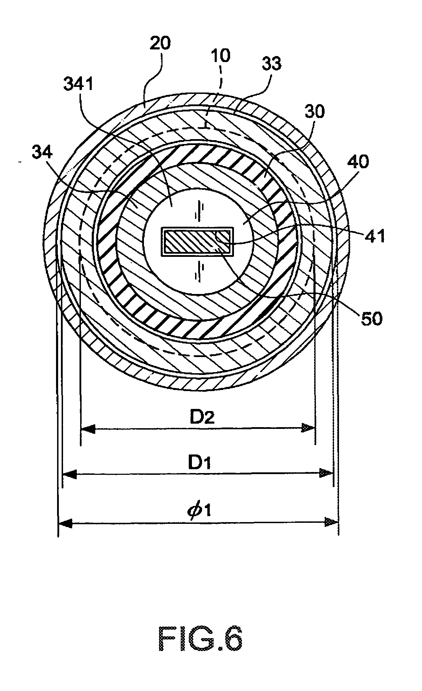

However, many tests done for a long time on the above-mentioned structure show that the internal rod 10 is lifted and rotated within the external rod 20 at the time when the external rod 20 is pushed downward and pulled upward (see FIGS. 5 and 6). The reason for that is that a tremendous frictional resistance tends to be created when the internal rod 10 is positioned too closely to the external rod 20, and when the external diameter D2 of the internal rod 10 is almost the same to the internal diameter φ1 of the external rod 20. In this way, the telescopic motion of the internal and external rods 10, 20 is not smooth and requires a great effort. If the gap between the internal and external rods 10, 20 is enlarged to eliminate the above-mentioned drawback, they would be placed in an unstable state and moved in a rocking and sloping way. Even an undesirably great noise can be produced. This requires further improvements.

In order to resolve the above-mentioned problems, the structure in accordance with the invention is provided with following features.

As shown in FIG. 6, the engaging element 30 is constructed as a cylindrical body with the middle and lower parts secured to the inside of the internal rod 10. The annular element 33 rotatable clockwise and counterclockwise at 360° is mounted on the top portion of the engaging element 30 projecting in an exposed manner from the internal rod 10. The external diameter D1 of the annular element 33 is greater than the external diameter D2 of the internal rod 10, but smaller than is almost the same to the internal diameter φ1 of the external rod 20. As shown in FIG. 5, the length L1 of the actuating element 40 is smaller than the length L2 of the inside of the engaging element 30. In addition, the bottom of the fixing cap 34 is extended and secured to the opening 32 of the engaging element 30 in such a way that a gap S is provided between the fixing cap 34 and the top of the actuating element 40. The fixing cap 34 includes at the top thereof the projecting flange 342 (whose external diameter is greater than the external diameter D2 of the internal rod 10, but smaller than the external diameter D1 of the annular element 33), for positioning the annular element 33 on the periphery of the top portion of the engaging element 30 without affecting the rotation of the annular element 33 within the external rod 20.

Furthermore, the driving element 50 includes at the bottom thereof a position-limiting element 52 and a positioning element 53 for a reliable stop of the driving element 50 in a preset position, and for a practical protection of the driving element 50 from being detached from the internal rod 10.

The structure in accordance with the invention is provided to resolve the problems with respect to the telescopic and rotary motions of the internal and external rods 10, 20. Moreover, the structure permits a more smooth operation with less effort. Meanwhile, the noise may be reduced and the service life may be increased.

## Claims

1. A telescopically rotatable mop, comprising:
a) an internal rod (10) having a hollow body;
b) an external rod (20) having a hollow body with a bottom portion in a telescopic connection with a top portion of the internal rod (10);
**characterized in that** it further comprises:
c) an engaging element (30), positioned within an opening (11) at the top of the internal rod (10), the engaging element (30) having at the bottom thereof a through hole (36) and at the internal bottom rim a plurality of upward driven teeth (35);
d) a driving element (50) formed in an elongated shape, constructed as a threaded piece, and positioned within the external rod (20) in such a way that the driving element (50) is moved up and down synchronically with the external rod (20), the driving element (50) including a fixing block (51) fastened within the top end (21) of the external rod (20) and the driving element (50) extending inside the internal rod (10);
e) an actuating element (40) positioned within the engaging element (30) with a threaded sleeve (41) at the top thereof for accommodating the driving element (50), the threaded sleeve (41) at the internal end of the actuating element (40) being constructed as an elongated groove such that the up-and-down movement of the driving element (50) in the threaded sleeve (41) may impart a rotary motion to the actuating element (40) within the engaging element (30), the actuating element (40) having at the bottom thereof a plurality of downward driving teeth (42) corresponding to the driven teeth (35) of the engaging element (30) for driving the engaging element (30) in a single direction when the actuating element (30) is rotated by the driving element (50), the driving teeth (42) and driven teeth (35) being formed in an inclined way;
f) a fixing cap (34) having a through hole (341) for the insertion of the driving element (50), the fixing cap (34) being mounted on an opening (32) of the engaging element (30);
g) a disc body (60) secured to the bottom of the internal rod (10) and having mop yarns (61);
h) a locking mechanism (70) mounted on the external rod (20) for locking the internal rod (10) and the external rod (20) in place or for unlocking them in a telescopic state;
wherein the engaging element (30) is constructed as a cylindrical body with the middle and lower parts secured to the inside of the internal rod (10), and an annular element (33) rotatable clockwise and counterclockwise at 360° is mounted on the periphery of the top portion of the engaging element (30) projecting in an exposed manner from the internal rod (10), the annular element (33) being within the external rod (20), and the external diameter D1 of the annular element (33) is greater than the external diameter D2 of the internal rod (10), but slightly smaller than the internal diameter φ1 of the external rod (20) ;
wherein the length L1 of the actuating element (40) is smaller than the length L2 of the inside of the engaging element (30);
wherein the bottom of the fixing cap (34) is extended and secured to the opening (32) of the engaging element (30) in such a way that a gap S is provided between the fixing cap (34) and the top of the actuating element (40), and the fixing cap (34) includes at the top thereof a projecting flange (342) whose external diameter is greater than the external diameter D2 of the internal rod (10) and smaller than the external diameter D1 of the annular element (33) for positioning the annular element (33) on the periphery of the top portion of the engaging element (30) without affecting the rotation of the annular element (33) within the external rod (20) ; and
wherein the locking mechanism (70) includes:
a) an internal clamping sleeve (70a) having an internal tube (71) at the top thereof, the bottom of the external rod (20) being introduced into the internal tube (71) and fastened there in place, both sides of the internal clamping sleeve (70a) being provided with positioning holes (72), the bottom portion of the internal clamping sleeve (70a) being constructed as a conic body (73) extending or expanding from the top of said bottom portion to the bottom of said bottom portion and having an indentation (74);
b) an external clamping sleeve (70b) being mounted on the periphery of the internal clamping sleeve (70a), the upper portion of the external clamping sleeve (70b) being provided with an external tube (75) corresponding to the internal tube (71), the external tube (75) having at both sides thereof two mounting holes (76) in alignment with the positioning holes (72) of the internal tube (71), a bell mouth (77) being formed at the lower portion of the external clamping sleeve (70b) for fitting over the conic body (73); and
c) a U-shaped lever (70c) having a swivel protrusion (79) and an eccentric cam (78) at the internal wall of both sides thereof for fitting into the positioning holes (72) of the internal clamping sleeve (70a) and the mounting holes (76) of the external clamping sleeve (70b), the eccentric cams (78) being positioned within the mounting holes (76);
whereby when the U-shaped lever (70c) swivels on the swivel protrusion (79), the eccentric cams (78) are offset within the mounting holes (76), thereby moving the external clamping sleeve (70b) on the periphery of the internal clamping sleeve (70a) upward or downward;
wherein, due to the action of the indentation, the bell mouth (77) of the external clamping sleeve (70b) is brought in a tightened or loosened position relative to the conic body (73) of the internal clamping sleeve (70a), thereby locking the internal and external rods (10), (20) in place or unlocking them in a telescopic state.

2. The mop as recited in claim 1 wherein the driving element (50) includes at the bottom thereof a position-limiting element (52) and a positioning element (53) for a reliable stop of the driving element (50) in a preset position, and for a practical protection of the driving element (50) from being detached from the internal rod (10).

3. The mop as recited in claim 1 wherein the engaging element (30) includes a flange (31) at the periphery of the top portion thereof for securing the engaging element (30) to the opening of the internal rod (10).

4. Mop assembly, comprising the telescopically rotatable mop as recited in any one of claims 1 to 3, wherein the assembly includes a dewatering basket (81) rotatable within a bucket body (80), so that the dewatering basket (81) is synchronically driven in rotation when the disc body (60) is rotated by the internal rod (10), whereby the mop yarns (61) of the disc body (60) are subject to the centrifugal force for dewatering, and the water removed may be received within the bucket body (80).

## Patentansprüche

1. Teleskopisch drehbarer Mopp, umfassend:
a) einen inneren Stab (10), der einen hohlen Körper aufweist;
b) einen äußeren Stab (20), der einen hohlen Körper mit einem unteren Abschnitt in einer Teleskopverbindung mit einem oberen Abschnitt des inneren Stabs (10) aufweist;
**dadurch gekennzeichnet, dass** er ferner Folgendes umfasst:
c) ein Eingriffelement (30), das in einer Öffnung (11) oben an dem inneren Stab (10) platziert ist, wobei das Eingriffelement (30) am Boden davon ein durchgehendes Loch (36) und an dem inneren unteren Rand eine Vielzahl von nach oben gerichteten angetriebenen Zähnen (35) aufweist;
d) ein Antriebselement (50), das in einer länglichen Form geformt ist, als Gewindestück aufgebaut ist und derart in dem äußeren Stab (20) platziert ist, dass das Antriebselement (50) synchron mit dem äußeren Stab (20) nach oben und nach unten bewegt wird, wobei das Antriebselement (50) einen Befestigungsblock (51) aufweist, der in dem oberen Ende (21) des äußeren Stabs (20) befestigt ist, und das Antriebselement (50) in dem inneren Stab (10) verläuft;
e) ein Betätigungselement (40), das in dem Eingriffelement (30) platziert ist, mit einer Gewindehülse (41) am Oberteil davon zum Aufnehmen des Antriebselements (50), wobei die Gewindehülse (41) am inneren Ende des Betätigungselements (40) als längliche Nut aufgebaut ist, sodass die Auf- und Abbewegung des Antriebselements (50) in der Gewindehülse (41) eine Drehbewegung auf das Betätigungselement (40) in dem Eingriffelement (30) übertragen kann, wobei das Betätigungselement (40) an der Unterseite davon eine Vielzahl von nach unten gerichteten Antriebszähnen (42) aufweist, die den angetriebenen Zähnen (35) des Eingriffelements (30) entsprechen, um das Eingriffelement (30) in eine einzige Richtung anzutreiben, wenn das Betätigungselement (30) von dem Antriebselement (50) gedreht wird, wobei die Antriebszähne (42) und angetriebenen Zähne (35) auf geneigte Weise geformt sind;
f) eine Befestigungskappe (34), die ein durchgehendes Loch (341) zum Einführen des Antriebselements (50) aufweist, wobei die Befestigungskappe (34) auf einer Öffnung (32) des Eingriffelements (30) montiert ist;
g) einen Plattenkörper (60), der unten an dem inneren Stab (10) befestigt ist und Moppfäden (61) aufweist;
h) einen Arretierungsmechanismus (70), der an dem äußeren Stab (20) montiert ist, um den inneren Stab (10) und den äußeren Stab (20) zu arretieren oder ihre Arretierung in einem Teleskopzustand zu lösen;
wobei das Eingriffelement (30) als zylindrischer Körper aufgebaut ist, wobei der mittlere und untere Teil an der Innenseite des inneren Stabs (10) gesichert sind, und ein ringförmiges Element (33), das um 360° in Uhrzeigerrichtung und entgegen der Uhrzeigerrichtung drehbar ist, am Umfang des oberen Abschnitts des Eingriffelements (30) montiert ist und auf freiliegende Weise von dem inneren Stab (10) vorsteht, wobei sich das ringförmige Element (33) innerhalb des äußeren Stabs (20) befindet und der Außendurchmesser D1 des ringförmigen Elements (33) größer ist als der Außendurchmesser D2 des inneren Stabs (10), jedoch etwas geringer als der Innendurchmesser Φ1 des äußeren Stabs (20) ;
wobei die Länge L1 des Betätigungselements (40) kürzer ist als die Länge L2 der Innenseite des Eingriffelements (30); wobei das Unterteil der Befestigungskappe (34) verlängert ist und an der Öffnung (32) des Eingriffelements (30) derart gesichert ist, dass ein Spalt S zwischen der Befestigungskappe (34) und der Oberseite des Betätigungselements (40) vorgesehen ist, und die Befestigungskappe (34) an der Oberseite davon einen vorstehenden Bund (342) aufweist, dessen Außendurchmesser größer ist als der Außendurchmesser D2 des inneren Stabs (10) und geringer als der Innendurchmesser D1 des ringförmigen Elements (33), um das ringförmige Element (33) an dem Umfang des oberen Abschnitts des Eingriffelements (30) zu platzieren, ohne die Drehung des ringförmigen Elements (33) in dem äußeren Stab (20) zu beeinträchtigen; und
wobei der Arretierungsmechanismus (70) Folgendes aufweist:
a) eine innere Klemmhülse (70a), die am oberen Teil davon eine innere Röhre (71) aufweist, wobei das Unterteil des äußeren Stabs (20) in die innere Röhre (71) eingeführt und dort befestigt ist, wobei beide Seiten der inneren Klemmhülse (70a) mit Positionierlöchern (72) versehen sind, wobei der untere Abschnitt der inneren Klemmhülse (70a) als kegelförmiger Körper (73) aufgebaut ist, der sich vom oberen Teil des unteren Abschnitts zum unteren Teil des unteren Abschnitts verbreitert oder erweitert und einen Einschnitt (74) aufweist;
b) eine äußere Klemmhülse (70b), die am Umfang der inneren Klemmhülse (70a) angebracht ist, wobei der obere Abschnitt der äußeren Klemmhülse (70b) mit einer äußeren Röhre (75) versehen ist, die der inneren Röhre (71) entspricht, wobei die äußere Röhre (75) an beiden Seiten davon zwei Montagelöcher (76) aufweist, die nach den Positionierlöchern (72) der inneren Röhre (71) ausgerichtet sind, wobei eine Aufweitung (77) am unteren Abschnitt der äußeren Klemmhülse (70b) gebildet ist, um über den kegelförmigen Körper (73) zu passen; und
c) einen U-förmigen Hebel (70c), der einen schwenkbaren Vorsprung (79) und einen außermittigen Ansatz (78) an der Innenwand beider Seiten davon aufweist, um in die Positionierlöcher (72) der inneren Klemmhülse (70a) und die Montagelöcher (76) der äußeren Klemmhülse (70b) zu passen, wobei die außermittigen Ansätze (78) in den Montagelöchern (76) platziert sind;
wodurch, wenn der U-förmige Hebel (70c) an dem schwenkbaren Vorsprung (79) geschwenkt wird, die außermittigen Ansätze (78) in den Montagelöchern (76) versetzt werden, wodurch die äußere Klemmhülse (70b) an dem Umfang der inneren Klemmhülse (70a) nach oben oder unten bewegt wird;
wobei, aufgrund der Wirkung des Einschnitts, die Aufweitung (77) der äußeren Klemmhülse (70b) bezüglich des kegelförmigen Körpers (73) der inneren Klemmhülse (70a) in eine gestraffte oder gelockerte Stellung gebracht wird, dadurch der innere und äußere Stab (10), (20) arretiert oder ihre Arretierung in einem Teleskopzustand gelöst wird.

2. Mopp nach Anspruch 1, wobei das Antriebselement (50) am unteren Teil davon ein Positionsbegrenzungselement (52) und ein Positionierelement (53) für einen zuverlässigen Stopp des Antriebselements (50) in einer voreingestellten Stellung und für einen praktischen Schutz des Antriebselements (50) davor, sich von dem inneren Stab (10) zu lösen, aufweist.

3. Mopp nach Anspruch 1, wobei das Eingriffelement (30) einen Bund (31) am Umfang des oberen Abschnitts davon zum Sichern des Eingriffelements (30) an der Öffnung des inneren Stabs (10) aufweist.

4. Moppanordnung, die den teleskopisch drehbaren Mopp nach einem der Ansprüche 1 bis 3 umfasst, wobei die Anordnung einen Entwässerungskorb (81) aufweist, der in einem Eimerkörper (80) drehbar ist, sodass der Entwässerungskorb (81) synchron in Drehung versetzt wird, wenn der Plattenkörper (60) von dem inneren Stab gedreht wird, wodurch die Moppfäden (61) des Plattenkörpers (60) zur Entwässerung der Fliehkraft unterliegen und das beseitigte Wasser in dem Eimerkörper (80) aufgenommen werden kann.

## Revendications

1. Serpillière pouvant tourner télescopiquement, comprenant :
a) une tige interne (10) ayant un corps creux ;
b) une tige externe (20) ayant un corps creux avec une partie inférieure en liaison télescopique avec une partie supérieure de la tige interne (10) ;
**caractérisée par le fait qu'**elle comprend en outre :
c) un élément d'engagement (30), positionné à l'intérieur d'une ouverture (11) à la partie supérieure de la tige interne (10), l'élément d'engagement (30) ayant, à la partie inférieure de celui-ci, un trou traversant (36) et à la bordure inférieure interne, une pluralité de dents vers le haut entraînées (35) ;
d) un élément d'entraînement (50) formé dans une forme allongée, construit sous la forme d'une pièce filetée, et positionné à l'intérieur de la tige externe (20) d'une manière telle que l'élément d'entraînement (50) est déplacé vers le haut et vers le bas de façon synchrone avec la tige externe (20), l'élément d'entraînement (50) comprenant un bloc de fixation (51) fixé à l'intérieur de l'extrémité supérieure (21) de la tige externe (20) et l'élément d'entraînement (50) s'étendant à l'intérieur de la tige interne (10) ;
e) un élément d'actionnement (40) positionné à l'intérieur de l'élément d'engagement (30) avec un manchon fileté (41) à la partie supérieure de celui-ci pour recevoir l'élément d'entraînement (50), le manchon fileté (41) à l'extrémité interne de l'élément d'actionnement (40) étant construit sous la forme d'une rainure allongée de telle sorte que le mouvement vers le haut et vers le bas de l'élément d'entraînement (50) dans le manchon fileté (41) peut communiquer un mouvement de rotation à l'élément d'actionnement (40) à l'intérieur de l'élément d'engagement (30), l'élément d'actionnement (40) ayant, à la partie inférieure de celui-ci, une pluralité de dents vers le bas d'entraînement (42) correspondant aux dents entraînées (35) de l'élément d'engagement (30) pour entraîner l'élément d'engagement (30) dans un seul sens lorsque l'élément d'actionnement (30) est amené à tourner par l'élément d'entraînement (50), les dents d'entraînement (42) et les dents entraînées (35) étant formées d'une manière inclinée ;
f) un capuchon de fixation (34) ayant un trou traversant (341) pour l'introduction de l'élément d'entraînement (50), le capuchon de fixation (34) étant monté sur une ouverture (32) de l'élément d'engagement (30) ;
g) un corps en disque (60) fixé à la partie inférieure de la tige interne (10) et ayant des franges de serpillière (61) ;
h) un mécanisme de verrouillage (70) monté sur la tige externe (20) pour verrouiller la tige interne (10) et la tige externe (20) en place ou pour les déverrouiller dans un état télescopique ;
l'élément d'engagement (30) étant construit sous la forme d'un corps cylindrique avec les parties moyenne et inférieure fixées à l'intérieur de la tige interne (10), et un élément annulaire (33) pouvant tourner dans le sens des aiguilles d'une montre et dans le sens contraire des aiguilles d'une montre à 360° étant monté sur la périphérie de la partie supérieure de l'élément d'engagement (30) se projetant d'une manière exposée à partir de la tige interne (10), l'élément annulaire (33) se situant à l'intérieur de la tige externe (20), et le diamètre externe D1 de l'élément annulaire (33) étant supérieur au diamètre externe D2 de la tige interne (10), mais légèrement inférieur au diamètre interne ϕ₁ de la tige externe (20) ; la longueur L1 de l'élément d'actionnement (40) étant inférieure à la longueur L2 de l'intérieur de l'élément d'engagement (30) ;
la partie inférieure du capuchon de fixation (34) étant étendue et fixée à l'ouverture (32) de l'élément d'engagement (30) d'une manière telle qu'un intervalle S est disposé entre le capuchon de fixation (34) et la partie supérieure de l'élément d'actionnement (40), et le capuchon de fixation (34) comprend à la partie supérieure de celui-ci une bride en saillie (342) dont le diamètre externe est supérieur au diamètre externe D2 de la tige interne (10) et inférieur au diamètre externe D1 de l'élément annulaire (33) pour un positionnement de l'élément annulaire (33) sur la périphérie de la partie supérieure de l'élément d'engagement (30) sans affecter la rotation de l'élément annulaire (33) à l'intérieur de la tige externe (20) ; et le mécanisme de verrouillage (70) comprenant :
a) un manchon de verrouillage interne (70a) ayant un tube interne (71) à la partie supérieure de celui-ci, la partie inférieure de la tige externe (20) étant introduite dans le tube interne (71) et fixée là en place, les deux côtés du manchon de serrage interne (70a) comportant des trous de positionnement (72), la partie inférieure du manchon de serrage interne (70a) étant construite en tant que corps conique (73) s'étendant ou s'expansant à partir de la partie supérieure de ladite partie inférieure à la partie inférieure de ladite partie inférieure et ayant une indentation (74) ;
b) un manchon de serrage externe (70b) étant monté sur la périphérie du manchon de serrage interne (70a), la partie supérieure du manchon de serrage externe (70b) comportant un tube externe (75) correspondant au tube interne (71), le tube externe (75) ayant des deux côtés de celui-ci deux trous de montage (76) en alignement avec les trous de positionnement (72) du tube interne (71), un évasement doucine (77) étant formé à la partie inférieure du manchon de serrage externe (70b) pour adaptation sur le corps conique (73) ; et
c) un levier en forme de U (70c) ayant une saillie de pivotement (79) et une came excentrique (78) à la paroi interne de ses deux côtés pour s'adapter dans les trous de positionnement (72) du manchon de serrage interne (70a) et les trous de montage (76) du manchon de serrage externe (70b), les cames excentriques (78) étant positionnées à l'intérieur des trous de montage (76) ;
ce par quoi, lorsque le levier en forme de U (70c) pivote sur la saillie de pivotement (79), les cames excentriques (78) sont décalées à l'intérieur des trous de montage (76), permettant ainsi de déplacer le manchon de serrage externe (70b) sur la périphérie du manchon de serrage interne (70a) vers le haut ou vers le bas ;
où, en raison de l'action de l'indentation, l'évasement doucine (77) du manchon de serrage externe (70b) est amené dans une position serrée ou relâchée par rapport au corps conique (743) du manchon de serrage interne (70a), verrouillant ainsi les tiges interne et externe (10), (20) en place ou les déverrouillant dans un état télescopique.

2. Serpillière selon la revendication 1, dans laquelle l'élément d'entraînement (50) comprend à la partie inférieure de celui-ci un élément de limitation de position (52) et un élément de positionnement (53) pour un arrêt fiable de l'élément d'entraînement (50) dans une position préfixée, et pour une protection pratique de l'élément d'entraînement (50) vis-à-vis d'un détachement de la tige interne (10).

3. Serpillière selon la revendication 1, dans laquelle l'élément d'engagement (30) comprend une bride (31) à la périphérie de la partie supérieure de celui-ci pour fixer l'élément d'engagement (30) sur l'ouverture de la tige interne (10).

4. Ensemble comprenant la serpillière pouvant tourner de façon télescopique selon l'une quelconque des revendications 1 à 3, dans lequel l'ensemble comprend un panier d'essorage (81) pouvant tourner à l'intérieur d'un corps de baquet (80), de telle sorte que le panier d'essorage (81) est entraîné en rotation de façon synchrone lorsque le corps de disque (60) est amené à tourner par la tige interne (10), ce par quoi les franges de serpillière (61) du corps de disque (60) sont soumises à une force centrifuge pour l'essorage, et l'eau retirée peut être reçue à l'intérieur du corps de baquet (80).
